# EUROPEAN PATENT SPECIFICATION

(11) **EP 0 295 893 B1**
(45) Date of publication and mention of the grant of the patent: **21.09.1994**
(21) Application number: 88305467.8
(22) Date of filing: 15.06.1988
(51) Int. Cl.: G01S 7/52, A61B 8/00

(54) **Ultrasonic analyzers**
Ultraschall-Analysatoren
Analyseurs d'ultrasons

(30) Priority: 15.06.1987 JP 148406/87; 15.06.1987 JP 148407/87; 19.06.1987 JP 152923/87
(43) Date of publication of application: 21.12.1988
(73) Proprietor: FUJITSU LIMITED, Kawasaki-shi, Kanagawa 211 (JP)
(72) Inventor: Shiba, Akira, Miyamae-ku Kawasaki-shi Kanagawa 213-54 (JP); Yamada, Isamu, Takatsu-ku Kawasaki-shi Kanagawa 213 (JP); Murakami, Keiichi, Kawasaki-shi Kanagawa 214 (JP)
(74) Representative: Fane, Christopher Robin King

(56) References cited:
- EP-A- 0 166 836
- US-A- 4 512 195
- US-A- 4 574 635

## Description

The present invention relates to ultrasonic analyzers.

Ultrasonic diagnostic instruments are widely utilized in the field of medicine. In use, such an instrument emits ultrasonic pulses which are reflected by a medium, such a living body, to be investigated, and measures an acoustic characteristic, for example an attenuation coefficient, of the reflected pulses (echo signals). An ultrasonic analyzer is used for analyzing the acoustic characteristic of such echo signals which are received as time-series signals. In general, the echo signals contain a significant proportion of noise, caused by pulses being reflected from various complex obstacles in the medium, such as complex blood vessels in the living body for example in order to obtain a precise detection result when measuring the acoustic characteristic, it is necessary to eliminate such noises from the echo signals or avoid such noises when analyzing the echo signals.

Accordingly it is desirable to provide an ultrasonic analyzer which may be used in an ultrasonic diagnosis instrument and which can enable high precision measurement of an acoustic characteristic of an echo signal reflected from a medium.

According to an embodiment of the present invention which is defined by claim 1 and 11, there is provided an ultrasonic analyzer, for use in an ultrasonic instrument operable to direct ultrasonic pulses at a medium to be studied and to detect an echo signal y(t) of those pulses reflected from said medium, in which instrument an analysis selection signal w(t) is generated in dependence upon the detected echo signal y(t) such that the analysis selection signal w(t) has a first value during the periods when the echo signal y(t) contains noise components and a second value outside those periods, which analyzer is operable to determine the coefficient a₁ of a regression function f(t)=a₁t + a₀ approximating to the echo signal y(t) detected during an analysis interval of duration T centred on a selected analysis time measured relative to a reference time and comprises:
first means, connected to receive said analysis selection signal w(t) and said echo signal y(t), for producing respective output signals indicative of the start time t₁ and end time t₂ of said analysis interval when an analysis time has been selected, which first means are operable during receipt of said echo signal y(t) to determine the total duration of successive periods when the echo signal y(t) does not contain noise components and to store successive values thereof with respect to the time t elapsed from said reference time, and to employ those values when an analysis time is selected to determine the start time t₁ and end time t₂ such that the total duration of such periods between t₁ and t and the total duration of such periods between t and t₂ are each equal to T/2;
second means connected to receive said analysis selection signal w(t) and said echo signal y(t) and operable, during receipt of said echo signal y(t), to employ those signals w(t), y(t) to generate a set of four integration signals calculated by accumulating respective multiplication signals w(t)t, w(t)t². w(t)y(t)t, w(t)y(t), which multiplication signals are produced by multiplying said analysis selection signal w(t) by respective ones of a set of multiplier signals consisting of a time signal t representing the time which has elapsed from the reference time, a square signal t² representing the square of the said time signal t, a product signal y(t)t representing the multiplication of the said echo signal y(t) by said time signal t, and the said echo signal y(t);
third means connected to receive the output signals of said first means, which third means are operable to store successive values of each of the said integration signals with respect to the time t elapsed from said reference time, and, upon receipt of said output signals, to provide at respective outputs thereof signals indicative of the difference between the stored values at the start and end times t₁, t₂ in respect of each of the said integration signals; and
fourth means connected to receive the signals provided at the said outputs of the third means and to employ those signals to calculate the coefficient a₁ of said regression function f(t) from the equation:-$\text{a₁ =} \frac{\text{TΣw(t)y(t)t - Σw(t)y(t).Σw(t)t}}{\text{TΣw(t)t² - ++(Σw(t)t)²}}$

Reference will now be made, by way of example, to the accompanying drawings; in which;
Figs. 1A and 1D illustrate a previously-proposed analysis of an echo signal;
Fig. 1B shows an actual waveform of an echo signal reflected from a media;
Fig. 1C shows a waveform of an analysis selection signal;
Fig. 2 is a schematic block diagram of an ultrasonic analyzer embodying the present invention.
Figs. 3A to 3E are graphs for use in explaining a method of analysis for use in an embodiment of the present invention;
Fig. 4 is a detailed block diagram of an analysis interval calculation circuit shown in Fig. 2;
Fig. 5 represents information stored in memories of the circuit of Fig. 4;
Fig. 6 shows a timing control circuit for generating various control signals for the circuitry shown in Fig. 2;
Fig 7 is a detailed block diagram of a running average calculation circuit shown in fig. 2;
Fig. 8 is a schematic block diagram of an analysis selection signal generation circuit embodying the present invention;
Fig. 9 is a detailed block diagram of the ultrasonic analyzer shown in Fig. 2; and
Fig. 10 is a schematic block diagram of another ultrasonic analyzer embodying the present invention.

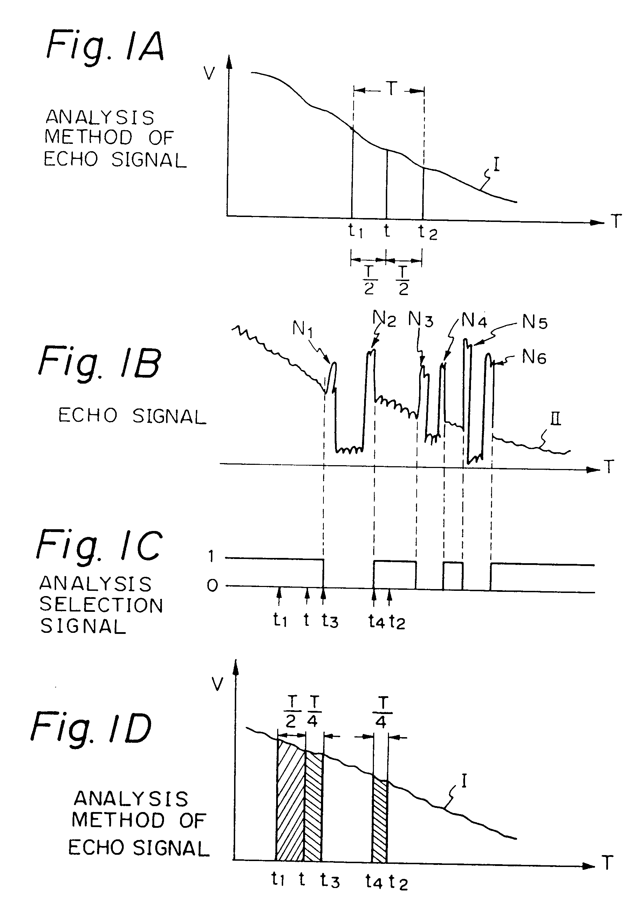

Before describing embodiments of the present invention, an explanation will be given, with reference to Figs. 1A and 1D, of a previously-considered method of analyzing the acoustic characteristic of an echo signal.

In Fig. 1A, a curve I represents an input signal to an ultrasonic analyzer, i.e. an echo signal reflected from a medium, such as a living body. This curve is obtained by an envelope of the echo signal after passing through a logarithm amplifier (not shown). In the Figure, the ordinate V is the signal level of the input signal and the abscissa T is the transition time of the input signal. Therefore, the input signal is given by the time-series signal and is analyzed in the analysis interval T. Each analysis interval has the same duration. The time t shows the central time of the analysis interval; the time t₁ shows a start point of the analysis, and the time t₂ shows an end point thereof. The intervals t-t₁ and t₂-t are each one half of the time of the analysis interval. The ultrasonic analyzer calculates the running average value at each analysis interval T and outputs the resultant data as a function of time. When pulse noise does not exist on the curve I, it is easy to obtain the start point t₁ and the end point t₂, since the analysis interval is constant. However, when pulse noises appear on the curve I, it is necessary to eliminate or omit such noise pulses from the analysis interval, to obtain the required data accurately.

In Fig. 1B, the curve II shows an actual waveform of the input signal (echo signal) containing various pulse noises N₁ to N₆. Such noise pulses are caused by reflection of the ultrasound pulses by various obstacles in the medium upon which the pulses are directed, for example blood vessels in a living body. These noise pulses also appear in the form of time-series signals.

In Fig. 1C an analysis selection signal is used for eliminating the noise intervals. When the analysis selection signal is at its high level ("1"), an analysis is made of the analysis interval T. When the analysis selection signal is low ("O"), the analysis is not made. If the analysis is performed regardless of the high/low level of the analysis selection signal, it is difficult to obtain an accurate result, since the resultant data contain noise components.

In Fig. 1D, the analysis interval is changed in such a way that the number of sampling data becomes constant in each analysis interval. That is, the number of sampling data in the analysis interval t-t₁ is equal to a sum of the intervals t₃-t and t₂-t₄. In this case, when the analysis selection signal is low, such an interval is not used for calculating the number of sampling data.

Nevertheless, many very complex calculations are necessary to obtain the data for each analysis interval, and, accordingly, much time is necessary for obtaining the acoustic characteristic of the living body and this delay reduces the speed of the diagnosis.

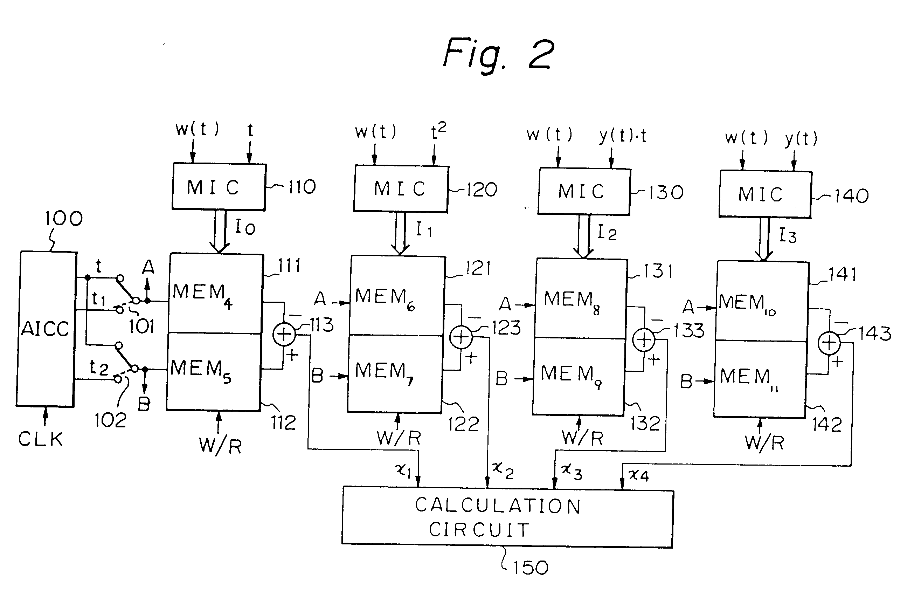

An ultrasonic analyzer embodying the present invention will now be explained with reference to Fig. 2.

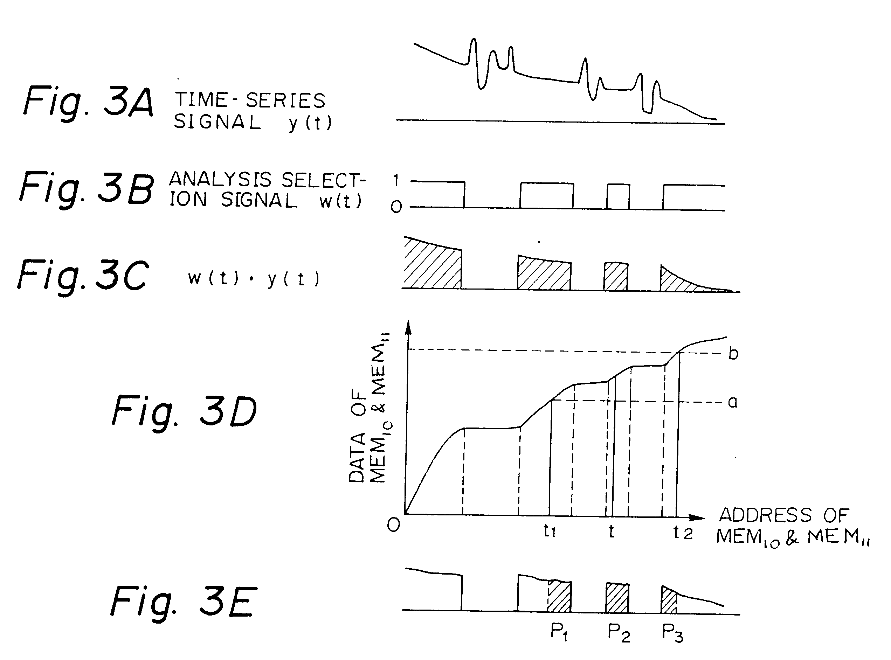

In Fig. 2, reference number 100 denotes an analysis interval calculation circuit (AICC), 110, 120, 130 and 140 are multiplication/integration circuits (MIC), 111, 121, 131 and 141 and 112, 122, 132 and 142 are memories (MEM₄, MEM₅,..), 113, 123, 133 and 143 are adders, and 150 is a calculation circuit. The MIC 110, the memories 111, 112 and the adder 113 constitute a running average calculation circuit (RACC). Similarly, the MIC 120, the memories 121, 122, and the adder 123, the MIC 130, the memories 131, 132 and the adder 133, and the MIC 140, the memories 141, 142, and the adder 143, constitute respective running average calculation circuits. The basic operation of this analyzer will be explained with reference to Figs. 2 and 3A to 3E.

In Fig. 2, the MIC 110 outputs an integration value I₀ obtained by multiplying the analysis selection signal w(t) by the time signal t. When switches 101 and 102 are located as shown in the drawing (solid line), the integration value I₀ is stored in the memories MEM₄ and MEM₅ in response to a write/read control signal (W/R). In this case, the time signal t is used as an address signal for storing the integration value I₀. Similarly, an integration value I₁ is obtained by multiplying the signal w(t) by a square signal t² of the time signal t in the MIC 120, and the integration value I₁ is stored in the memories MEM₆ and MEM₇; an integration value I₂ is obtained by multiplying the signal w(t) by a product signal y(t).t of the time-series signal y(t) and the time signal t in the MIC 130, and the integration value I₂ is stored in the memories MEM₈ and MEM₉; and an integration value I₃ is obtained by multiplying the signal w(t) by the time-series signal y(t) in the MIC 140, and the integration value I₃ is stored in the memories MEM₁₀ and MEM₁₁.

One example of the time-series signal y(t) is shown in Fig. 3A, and the analysis selection signal w(t) is shown in Fig. 3B. The product signal w(t).y(t) of the signal w(t) and the signal y(t) (i.e. integration value I₃) is shown in Fig. 3C. The product signal w(t).y(t) is obtained from the MIC 140. Figure 3D represents tabulated data stored in the memories MEM₁₀ and MEM₁₁. In Fig. 3D, the ordinate shows the data, and the abscissa shows the address. Every time that the integration value I₃ is stored in the memories MEM₁₀ and MEM₁₁, signal t is used as the address.

The switches 101 and 102 are located at opposite sides t₁ and t₂ (dotted line) after the above operations are finished. The analysis interval calculation circuit 100 outputs the start point t₁ and the end point t₂ of the analysis interval to all memories MEM₄ to MEM₁₁. In the drawing, the start point t₁ is shown by a signal A and the end point t₂ is shown by a signal B. Respective memories output a first integration value between the time "O" and the time "t₁", and a second integration value between the time "O" and the time "t₂". In Fig, 3D, the outputs a and b show the data read from the memories MEM₁₀ and MEM₁₁ at the start point t₁ and the end point t₂ respectively.

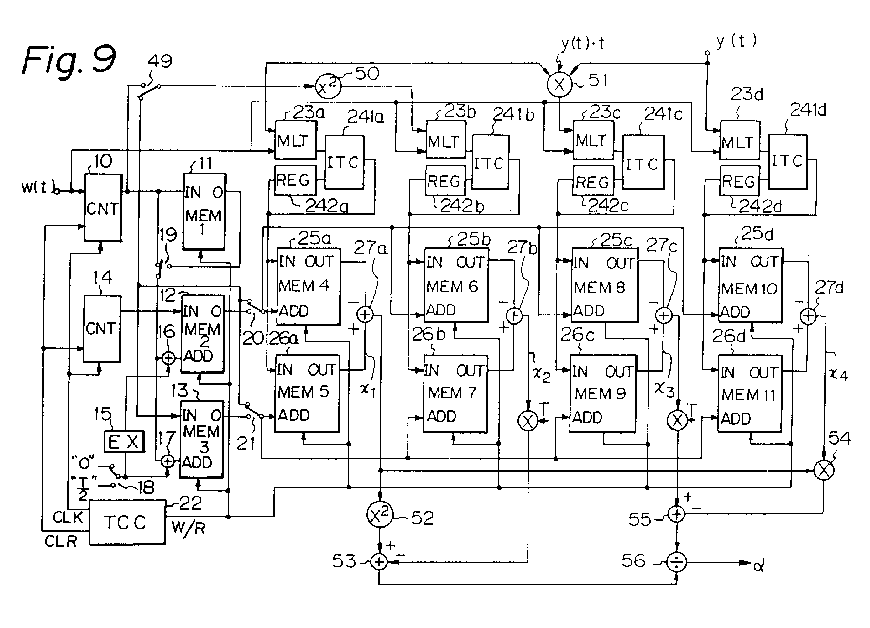

The difference between the first and the second integration values is calculated by the adders 113 to 143. For example, a difference value x₁ is obtained by the adder 113. That is, the adder 113 calculates the difference in the data of the memory MEM₄ and the memory MEM₅. Accordingly, the difference value x₁ means the integration value between the start point t₁ and the end point t₂. Similarly, the difference values x₂ and x₃ are the integration values between t₁ and t₂. The value x₄ from the adder 143 is shown in Fig. 3E. The difference value x₄ means the integration value between the start point t₁ and the end point t₂, and is equivalent to a sum of the slant-lined areas P₁, P₂, and P₃. The integration values x₁ to x₄ are input to the calculation circuit 150. The calculation circuit 150 is constituted by a plurality of adders and multipliers, and obtains a slope of a regression curve of the echo signal based on a least square method. The values x₁ to x₄ are explained in detail in Fig. 9.

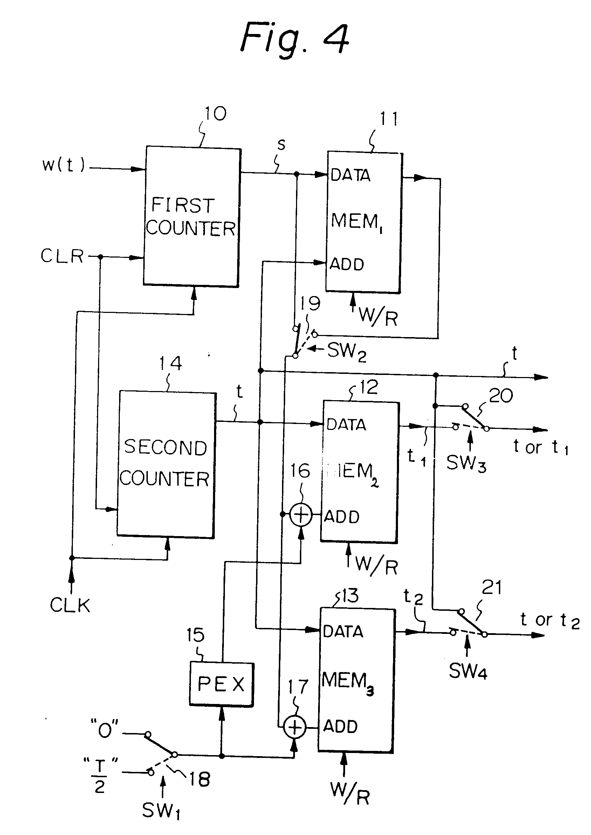

Figure 4 shows a detailed block diagram of the analysis interval calculation circuit 100 shown in Fig. 2. Fig. 4 shows a first counter circuit 10, memories 11 to 13 (MEM₁ to MEM₃), a second counter circuit 14, a polarity exchanger circuit 15, adders 16 and 17, and switches 18 to 21.

In a write mode, the switches 18 to 21 are located at the position shown in the drawing (solid lines). The analysis selection signal w(t) is input to an enable terminal of the first counter 10, and when the signal w(t) is high ("1"), the first counter 10 counts (integrates) a clock signal CLK, and the second counter 14 sequentially counts the clock signal CLK and outputs the time signal t. The write operation to each memory MEM₁ to MEM₃ is performed in response to the write/read control signal W/R. An integration signal S is input in parallel to the memories MEM₁ to MEM₃. In this case, the memory MEM₁ receives the integration signal S as the data (DATA), and the memories MEM₂ and MEM₃ receive the integration signal S as the address signal (ADD) through the adders 16 and 17. Further, the memory MEM₁ receives the time signal t as the address signal, and the memories MEM₂ and MEM₃ receive the time signal t as the data.

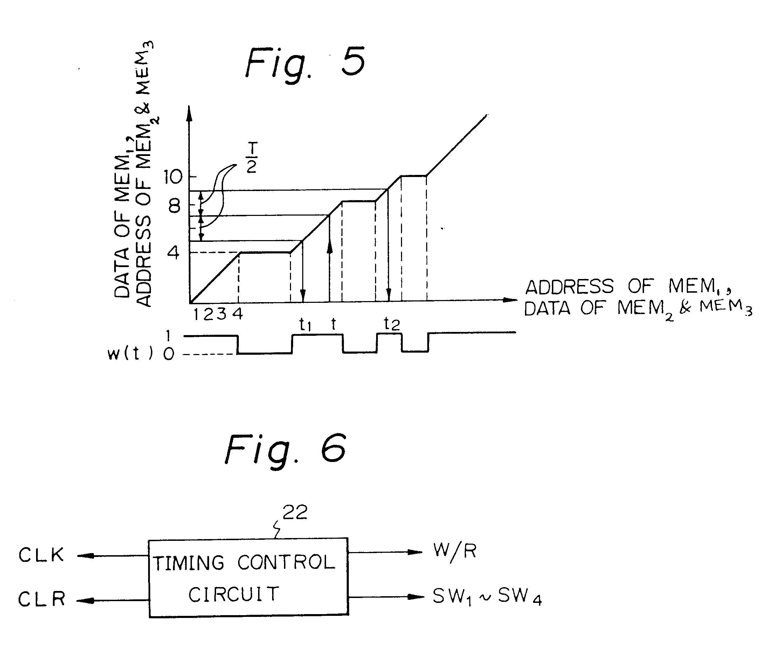

Figure 5 represents tabulated data stored in the memories MEM₁ to MEM₃. In Fig. 5, the ordinate denotes the data of the memory MEM₁ or the address of the memories MEM₂ and MEM₃, and the abscissa denotes the address of the memory MEM₁ or the data of the memories MEM₂ and MEM₃. As explained above, in the write mode, the memory MEM₁ stores the output signal S as the data in accordance with the address of the time signal t, and the memories MEM₂ and MEM₃ store the time signal t as the data in accordance with the address of the output signal S.

In a read mode, the switches 18 to 21 are located at the positions shown by dotted lines in Fig. 4. The start point t₁ and the end point t₂ are obtained in this mode. That is, when the write/read control signal W/R is input to each memory MEM₁ and MEM₃, the data stored in the memory MEM₁ is output to the memories MEM₂ and MEM₃ as the address signal through the switch 19 and the adders 16, 17. The data stored in memories MEM₂ and MEM₃ are output in accordance with the address from the memory MEM₁. In Fig. 5, the time data corresponding to the time interval T/2 is input to the adder 17 through the switch 18, the adder 17 adds the address at the time t to the address corresponding to the time interval T/2, and the memory MEM₃ is accessed by the address obtained by the output of the adder 17. The memory MEM₃ outputs the data indicating the end point t₂ as shown in Fig. 5. Similarly, the time data corresponding to the time interval T/2 is input to the adder 16 through the switch 18 and the polarity exchanger circuit 15. The adder 16 subtracts the address corresponding to the time interval T/2 from the address at the time t, and the memory MEM₂ outputs the data indicating the start point t₁, as shown in Fig. 5.

Figure 6 shows a timing control circuit. The timing control circuit 22 generates switch signals SW₁ to SW₄ for switching the contact points of the switches 18 to 21 in accordance with the write or read mode, a clear signal CLR for resetting the first counter 10 and the second counter 14, the clock signal CLK, and the write/read control signal W/R for controlling the write or read mode. The connection of the timing control circuit 22 to the other portions of the analyzer is shown in Fig. 9.

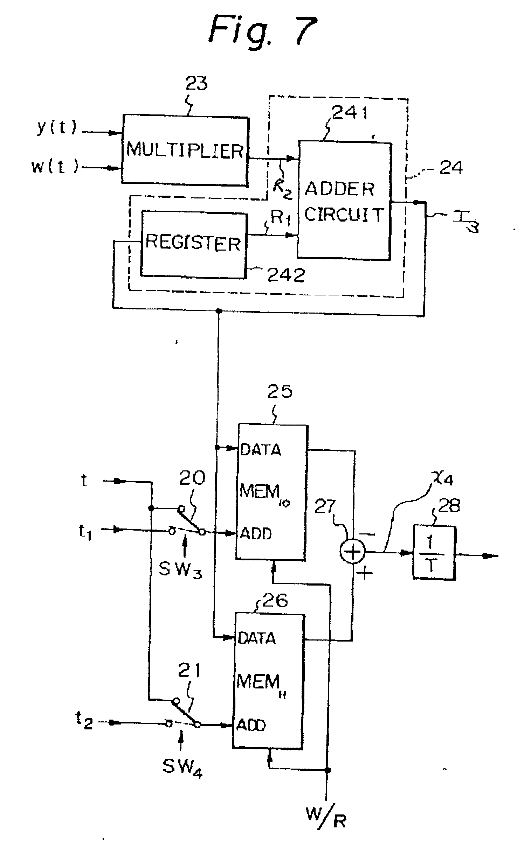

Figure 7 is a detailed block diagram of one of the running average circuits shown in Fig. 2. In Fig. 7, reference number 23 denotes a multiplication circuit for multiplying the time-series signal y(t) by the analysis selection signal w(t), 24 is an integration circuit consisting of an adder circuit 241 and a register 242, 25 and 26 are memories (MEM) corresponding to the memories MEM₁₀ and MEM₁₁ respectively of Fig. 2, 27 is an adder corresponding to the adder 143 of Fig. 2, and 28 is a divider circuit. The multiplication circuit 23 and the integration circuit 24 correspond to the multiplication integration circuit (MIC) 140 in Fig. 2.

A multiplication value R₂ is obtained by multiplying the time-series signal y(t) by the analysis selection signal w(t) in the multiplication circuit 23. These signals are shown in Figs. 3A to 3C. The adder circuit 241 adds the multiplication value R₂ to previous data R₁ provided by the register 242, and the resultant integration value I₃ is fed back to the register 242 and output to the memories 25 and 26. In the write mode, the integration value I₃ is stored in the memories 25 and 26 in accordance with the address of the time signal t transferred from the second counter 14 (Fig. 4) through the switch 20 and 21. Therefore, the memories 25 and 26 store the table shown in Fig. 3D.

In the read mode, the switches 20 and 21 are switched to the position shown by a dotted line. The start point t₁ and the end point t₂ are output from the analysis interval calculation circuit 100 shown in Fig. 4, and input to the memories 25 and 26 as the address, and the data stored in the memories 25 and 26 are output to the adder 27. The adder 27 calculates the difference between the data b at the time t₂ and the data a at the time t₁ as shown in Fig. 3D. The resultant data from the adder 27 is shown by P₁, P₂, and P₃ in Fig. 3E. The divider circuit 28 calculates the running average value. The running average value can be obtained by dividing the integration value (difference value) b-a by the analysis interval T. The analysis interval also denotes the number of the sampling data at the analysis interval T.

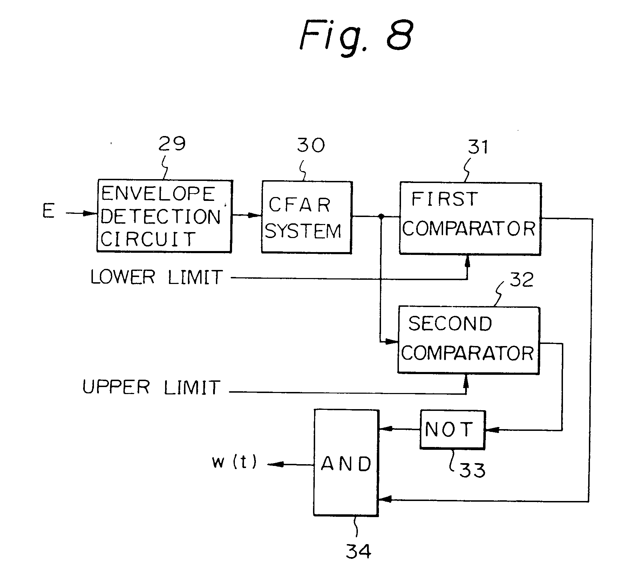

Figure 8 is a schematic block diagram of an analysis selection signal generation circuit embodying the present invention.

In Fig. 8, reference number 29 denotes an envelope detection circuit, 30 is a CFAR system, 31 and 32 are comparators, 33 is a NOT circuit, and 34 is an AND circuit. "E" denotes the echo signal reflected from the living body when the ultrasonic pulse is directed thereon. The echo signal E contains the various noise pulses N₁ to N₆ as shown in Fig. 1B. The envelope detection circuit 29 detects the envelope of the echo signal E and obtains a logarithm curve thereof. The CFAR system 30 applies a system employed in a radar system: "CFAR" means Constant False Alarm Rate. In general, the CFAR system is used for discriminating an unclear image contained in a reflected signal; an embodiment of the present invention utilizes the CFAR technique for detecting the various noises contained in the echo signal E. The CFAR system 30 generates a CFAR signal based on the logarithm curve of the envelope. The CFAR signal contains various noise pulses, but the first comparator 31 cuts out large dip noises which exceed a lower limit level, and the second comparator 32 cuts out large peak noises which exceed an upper limit level. The output of the comparator 32 is inverted by the NOT circuit 33, and, therefore, the analysis selection signal w(t) can be obtained from the AND circuit 34. That is, when the CFAR signal exceeds these limit lines, the analysis selection signal w(t) becomes low level ("0"), and when the CFAR signal is between the upper limit and the lower limit, the analysis selection signal w(t) becomes high level ("1"). The waveform of the signal w(t) is shown in Fig. 3B.

The feature of the analysis selection signal generation circuit lies in the utilization of the CFAR system as explained above. Previously-proposed analysis selection signal generation circuits do not utilize the CFAR system.

Figure 9 is a detailed block diagram of the ultrasonic analyzer shown in Fig. 2.

In Fig. 9: 23a to 23d denote multiplication circuits (MLT); 241a to 241d are integration circuits (ITC); 242a to 242d are registers (REG); 25a to 25d and 26a to 26d are memories (MEM₄ to MEM₁₁); 27a to 27d are adders; 10 and 14 are counters; 11 to 13 are memories (MEM₁ to MEM₃); 15 is a polarity exchanger circuit (EX); 22 is a timing control circuit (TCC); 16, 17, 53 and 55 are adders; 18, 19, 20, 21 and 49 are switches; 50 and 52 are squaring circuits; 51 and 54 are multiplication circuits; and 56 is a divider circuit.

The multiplication circuit 23a, the integration circuit 241a, and the register 242a correspond to the multiplication integration circuit 110 shown in Fig. 2, and the memories 25a and 26a correspond to the respective memories 111 and 112 shown in Fig. 2, and so on. Further, the counters 10 and 14, the memories 11 to 13, the polarity exchanger circuit 15, and the timing control circuit 22 correspond to the analysis interval calculation circuit 100 shown in Fig. 2. Still further, the squaring circuit 52, the adders 53 and 55, the multiplier 54, and the divider circuit 56 correspond to the calculation circuit 150 in Fig. 2.

The circuitry of Fig. 9 is intended for the purpose of determining a slope α of the regression curve of the echo signal by using the least square method. The attenuation coefficient is one of the acoustic characteristics contained in the echo signal, as explained above, and in general, it is possible to diagnose medical conditions occurring inside a living body by checking the slope of the echo signal. The more precise the value obtained for the slope α, the more accurate will be the diagnosis.

The ultrasonic analyzer shown in Fig. 9 can obtain a specific function f(t) based on the analysis selection signal w(t) and the time-series signal y(t). The specific function f(t) having n-order is:${\text{f(t) = a}}_{\text{n}} {\text{t}}^{\text{n}} {\text{+ a}}_{\text{n-1}} {\text{t}}^{\text{n-1}} \text{+ .... + a₁t + a₀}$
where the factors aₙ, aₙ₋₁... a₁, a₀ denote the slope α and are output from the calculation circuit 150.

When the frequency spectrum of the echo signal has a Gaussian distribution, the primary function is suitable for the specific function as the regression curve.

That is, the specific function f(t) may be simplified to:$\text{f(t) = a₁t + a₀}$
As is obvious, the formula (2) denotes a linear function and the factor a₁ denotes the slope α.

The factors a₁ and a₀ can be obtained by the known linear regression method, as follows:$\text{a₁ =} \frac{\text{TΣw(t)y(t)t - Σw(t)y(t)·Σw(t)t}}{\text{TΣw(t)t² - (Σw(t)t)²}}$$\text{a₀ =} \frac{\text{Σw(t)y(t)·Σw(t)t - Σw(t)y(t)t·Σw(t)t}}{\text{TΣw(t)t² - (Σw(t)t)²}}$

The value "TΣw(t)t" can be obtained from the adder 27a, the value "TΣw(t)t²" from the adder 27b, the value "TΣw(t)y(t)t" from the adder 27c, and the value "TΣw(t)y(t)" from the adder 27d.

The squaring circuit 52 calculates the value (Σw(t)t²)², the adder 53 calculates the value (Σw(t)t²)² - Σw(t)t², and the multiplier 54 calculates the value (Σw(t)t)(Σw(t)y(t)). Accordingly, the divider circuit 56 calculates the formula (1) and can obtain the factor a₁ as the slope α.

In this case, the squaring circuit 50 calculates the value "t²" based on the time signal t from the second counter 14, The multiplication circuit 51 calculates the value "y(t)t" based on the time signal t and the time-series signal.

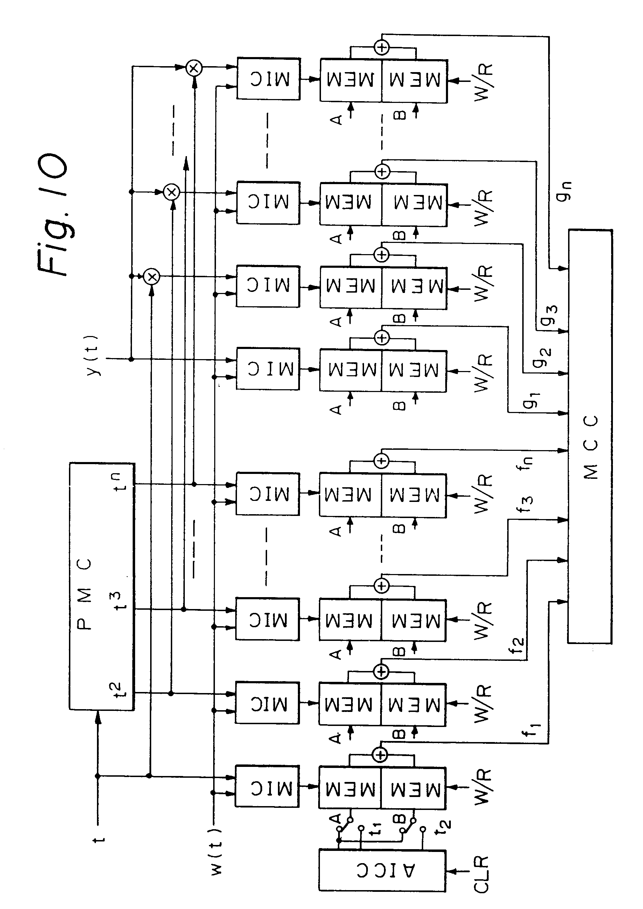

Figure 10 is a schematic block diagram of another ultrasonic analyzer embodying the present invention. Whilst the embodiment shown in Figs. 2 to 9 is for a case where the primary function is used as the specific function (see formula (2)), the embodiment of Fig. 10 is for a case where the n-order function is used as the specific function.

In Fig. 10, PMC denotes a power multiplication circuit for outputting power signals (t², t³ ..... tⁿ) of the time signal t. Each of these power signals is input to a corresponding multiplication integration circuit (MIC). Each of the product signals of the time-series signal y(t) and the power signal (t², t³ ..... tⁿ) is input to the corresponding multiplication integration circuit. In addition, the analysis selection signal w(t) is input in parallel to all of the MIC'S. MCC denotes a matrix calculation circuit for calculating the matrix given by formula (5) below.

That is, from the formula (1), a sum of the regression square error ER is: 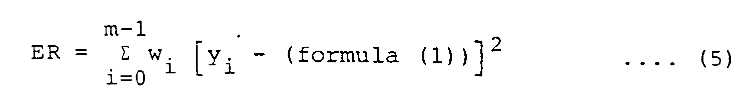
where "yᵢ" is an input signal as a discrete time signal at the time tᵢ. That is, the value of the input signal (y₀, y₁, y₂ .....) denotes the value corresponding to the time (t₀, t₁, t₃ .....).

The factors (aₙ, aₙ₋₁, ..... a₀) are determined from the minimum value of the regression square error ER.

For obtaining the minimum value, the following formula is used:$\frac{\text{∂ER}}{{\text{∂a}}_{\text{p}}} \text{= 0 (p = 0, 1, ....., n)}$

The formula (6) is given by the following matrix:

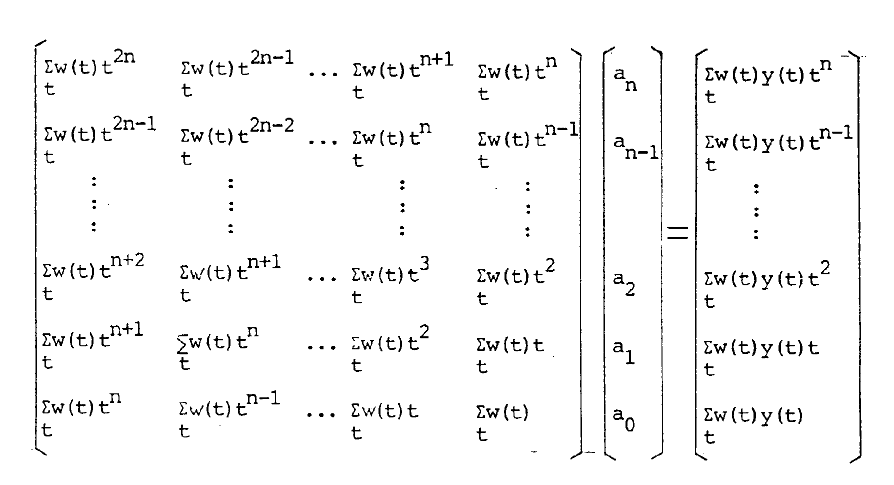

The factors (aₙ, aₙ₋₁, ....., a₀) can be obtained by solving the above matrix. Therefore, in the case of the n-order function, it is necessary to obtain the integration value of the formula w(t)y(t)t^{k} and w(t)t^{k}(k = 0 to n) for obtaining the above factors. The slope α of the n-order function can be obtained by differentiating the n-order function in the matrix calculation circuit.

Each integration value in Fig. 10 is given as follows: f₁ = Σw(t)t, f₂ = Σw(t)t², f₃ = Σw(t)t³, fₙ = Σw(t)tⁿ, g₁ = Σw(t)y(t), g₂ = Σw(t)y(t)t, g₃ = Σw(t)y(t)t², gₙ = Σw(t)y(t)tⁿ. In the above calculation, the analysis interval T is included in each calculation.

## Claims

1. An ultrasonic analyzer, for use in an ultrasonic instrument operable to direct ultrasonic pulses at a medium to be studied and to detect an echo signal y(t) of those pulses reflected from said medium, in which instrument an analysis selection signal w(t) is generated in dependence upon the detected echo signal y(t) such that the analysis selection signal w(t) has a first value during the periods when the echo signal y(t) contains noise components and a second value outside those periods, which analyzer is operable to determine the coefficient a₁ of a regression function f(t)=a₁t + a₀ approximating to the echo signal y(t) detected during an analysis interval of duration T centred on a selected analysis time measured relative to a reference time and comprises:
first means (100), connected to receive said analysis selection signal w(t) and said echo signal y(t), for producing respective output signals (A, B) indicative of the start time t₁ and end time t₂ of said analysis interval when an analysis time has been selected, which first means (100) are operable during receipt of said echo signal y(t) to determine the total duration of successive periods when the echo signal y(t) does not contain noise components and to store successive values (s) thereof with respect to the time t elapsed from said reference time, and to employ those values when an analysis time is selected to determine the start time t₁ and end time t₂ such that the total duration of such periods between t₁ and t and the total duration of such periods between t and t₂ are each equal to T/2;
second means (110, 120, 130, 140) connected to receive said analysis selection signal w(t) and said echo signal y(t) and operable, during receipt of said echo signal y(t), to employ those signals w(t), y(t) to generate a set of four integration signals (I₀, I₁, I₂, I₃) calculated by accumulating respective multiplication signals w(t)t, w(t)t², w(t)y(t)t, w(t)y(t), which multiplication signals are produced by multiplying said analysis selection signal w(t) by respective ones of a set of multiplier signals consisting of a time signal t representing the time which has elapsed from the reference time, a square signal t² representing the square, of the said time signal t, a product signal y(t)t representing the multiplication of the said echo signal y(t) by said time signal t, and the said echo signal y(t);
third means (111, 121, 131, 141) connected to receive the output signals (A, B) of said first means (100), which third means (111, 121, 131, 141) are operable to store successive values of each of the said integration signals (I₀, I₁, I₂, I₃) with respect to the time t elapsed from said reference time, and, upon receipt of said output signals (A, B), to provide at respective outputs (113, 123, 133, 143) thereof signals (x₁, x₂, x₃, x₄) indicative of the difference between the stored values at the start and end times t₁, t₂ in respect of each of the said integration signals (I₀, I₁, I₂, I₃); and
fourth means (150) connected to receive the signals (x₁, x₂, x₃, x₄) provided at the said outputs (113, 123, 133, 143) of the third means (111, 121, 131, 141) and to employ those signals to calculate the coefficient a₁ of said regression function f(t) from the equation:-$\text{a₁ =} \frac{\text{TΣw(t)y(t)t - Σw(t)y(t).Σw(t)t}}{\text{tΣw(t)t² - (Σw(t)t)²}}$

2. An ultrasonic analyzer as claimed in claim 1, wherein said first means (100) comprise: a first counter (10) for counting a clock signal (CLK) when said analysis selection signal w(t) has said second value, and for outputting an auxiliary integration signal (s) in response to said clock signal (CLK) during a write mode of the analyzer; a second counter (14) for counting said clock signal (CLK) and for outputting said time signal t; a first memory (11) for storing said auxiliary integration signal (s) by using said time signal t as an address; second and third memories (12, 13) for storing said time signal t as data by using said auxiliary integration signal (s) as the address; and first and second adder circuits (16, 17) for outputting respective first and second addresses (s-T/2; s+T/2) by shifting an address (s) output by said first memory (11) during a read mode of the analyzer by an address corresponding to T/2; said second memory (12) outputting said start time t₁ in response said first address (s-T/2) and said third memory (13) outputting said end time t₂ in response to said second address (s+T/2).

3. An ultrasonic analyzer as claimed in claim 2, further comprising a timing control circuit (22) for generating: said clock signal (CLK); a write/read control signal (W/R) for switching between said write mode and read mode, said write/read control signal (W/R) being input in parallel to said third means (111, 121, 131, 141); and a clear signal (CLR) for resetting said first and second counters (10, 14).

4. An ultrasonic analyzer as claimed in claim 2 or 3, wherein each of said first, second and third memories (11, 12, 13) comprises a table for storing said auxiliary integration signal (s) and said time signal t, said table having the address (t) of said first memory (11) or the data (t) of said second and third memories (12, 13) in an abscissa direction, and having the data (s) of said first memory (11) or the address (s) of said second and third memories (12, 13) in an ordinate direction.

5. An ultrasonic analyzer as claimed in any preceding claim, wherein said second means (110, 120, 130, 140) comprise four multiplication/integration circuits, for producing respective associated ones of said integration signals (I₀, I₁, I₂, I₃), each of which circuits comprises: a multiplication circuit (23) for multiplying said analysis selection signal w(t) by an associated one of said multiplier signals and outputting a multiplication value (R₂); an adder (241) circuit for integrating said multiplication value (R₂) and outputting said integration signal (I₀, I₁, I₂, I₃); and a register (242) for temporarily storing said integration signal (I₀, I₁, I₂, I₃).

6. An ultrasonic analyzer as claimed in any preceding claim, wherein said third means (111, 121, 131, 141) comprise four storage units for storing successive values of respective associated ones of the said integration signals (I₀, I₁, I₂, I₃), each storage unit comprising;
first and second memories (25, 26) connected to said first means (100) so as to receive at respective address inputs thereof the said time signal t during a write mode of the analyzer and respective ones of said output signals (A,B) during a read mode of the analyzer, the said first and second memories (25, 26) being operable during the write mode to store successive values of the integration signal (I₀, I₁, I₂, I₃) associated therewith at the addresses provided at their address inputs, and in a read mode to output respective items of such data stored at the addresses provided at their address inputs;
an adder circuit (27) connected to receive the data autput by said first and second memories (25, 26) and operable during said read mode to generate a signal (x₁, x₂, x₃, x₄) indicative of a difference value therebetween; and
a divider circuit (28) for calculating a running average value by dividing said difference value (x₁, x₂, x₃, x₄) by the duration of said analysis interval T.

7. An ultrasonic analyzer as claimed in claim 6, wherein each of said first and second memories (25, 26) comprises a table for storing said values of the integration signal (I₀, I₁, I₂, I₃), said table having the address of said first and second memories (25, 26) in an abscissa direction, and having the data of said first and second memories (25, 26) in an ordinate direction.

8. An ultrasonic analyzer as claimed in any preceding claim, wherein the signals (x₁, x₂, x₃, x₄) supplied to said fourth means (150) by said third means (111, 121, 131, 141) are indicative of first, second, third and fourth difference values (x₁, x₂, x₃, x₄) and said fourth means (150) comprise: a squaring circuit (52) for calculating a square value of said first difference value (x₁); a multiplication circuit (54) for multiplying said first difference value (x₁) by said fourth difference value (x₄); a first adder (53) for adding an output of said squaring circuit (52) to said second difference value (x₂); a second adder (55) for adding an output of said multiplication circuit (54) to said third difference value (x₃); and a divider circuit (56) for calculating the coefficient a₁ of said regression function f(t) by dividing an output of said second adder (55) by an output of said first adder (53).

9. An ultrasonic analyzer as claimed in any preceding claim, further comprising a squaring circuit (50) for generating said square signal t² from said time signal t, and a multiplication circuit (51) for generating said product signal y(t)t by multiplying said time signal t by said echo signal y(t).

10. An ultrasonic analyzer as claimed in any preceding claim, wherein said analysis selection signal w(t) is generated by an analysis selection signal generation circuit comprising: an envelope detection circuit (29) for obtaining an envelope from said echo signal y(t), and obtaining a logarithm curve from said envelope; a CFAR system (30) for generating a CFAR signal based on said logarithm curve; a first comparator (31) for comparing said CFAR signal with a lower limit level; a second comparator (32) for comparing said CFAR signal with an upper limit level; a NOT circuit (33) for inverting an output of said second comparator (32); and an AND circuit (34) for generating said analysis selection signal based on an output of said NOT circuit (33) and an output of said first comparator (31).

11. An ultrasonic analyzer, for use in an ultrasonic instrument operable to direct ultrasonic pulses at a medium to be studied and to detect an echo signal y(t) of those pulses reflected from said medium, in which instrument an analysis selection signal w(t) is generated in dependence upon the detected echo signal y(t) such that the analysis selection signal w(t) has a first value during the periods when the echo signal y(t) contains noise components and a second value outside those periods, which analyzer is operable to determine the coefficients a₁ to aₙ of a regression function f(t)=aₙtⁿ + aₙ₋₁tⁿ⁻¹ + .... + a₁t + a₀ approximating to the echo signal y(t) detected during an analysis interval of duration T centred on a selected analysis time measured relative to a reference time and comprises:
first means (100), connected to receive said analysis selection signal w(t) and said echo signal y(t), for producing respective output signals (A, B) indicative of the start time t₁ and end time t₂ of said analysis interval when an analysis time has been selected, which first means (100) are operable during receipt of said echo signal y(t) to determine the total duration of successive periods when the echo signal y(t) does not contain noise components end to store successive values (s) thereof with respect to the time t elapsed from said reference time, and to employ those values (s) when an analysis time is selected to determine the start time t₁ and end time t₂ such that the total duration of such periods between t₁ and t and the total duration of such periods between t and t₂ are each equal to T/2;
second means (110, 120, 130, 140, ....) connected to receive said analysis selection signal w(t) and said echo signal y(t) and operable, during receipt of said echo signal y(t), to employ those signals w(t), y(t) to generate a set of integration signals (I₀, I₁, I₂, I₃, ...) calculated by accumulating respective multiplication signals, which multiplication signals are produced by multiplying said analysis selection signal w(t) by respective ones of a set of multiplier signals consisting of a time signal t representing the time which has elapsed from the reference time, power signals t^{k}, where k = 0 to n, representing the kth power of the said time signal t, a product signal y(t)t^{k} representing the multiplication of the said echo signal y(t) by said power signals t^{k}, and the said echo signal y(t);
third means (111, 121, 131, 141, ....) connected to receive the output signals (A, B) of said first means (100), which third means (111, 121, 131, 141, ....) are operable to store successive values of each of the said integration signals (I₀, I₁, I₂, I₃, ....) with respect to the time t elapsed from said reference time, and, upon receipt of said output signals (A, B), to provide at respective outputs (113, 123, 133, 143, ...) thereof signals (x₁, x₂, x₃, x₄, ....) indicative of the difference between the stored values at the start and end times t₁, t₂ in respect of each of the said integration signals (I₀, I₁, I₂, I₃, ....); and
fourth means (150) connected to receive the signals (x₁, x₂, x₃, x₄, ....) provided at the said outputs of the third means (111, 121, 131, 141, ....) and to employ those values to calculate the coefficients a₁ to aₙ of said regression function f(t) by solving the matrix:-

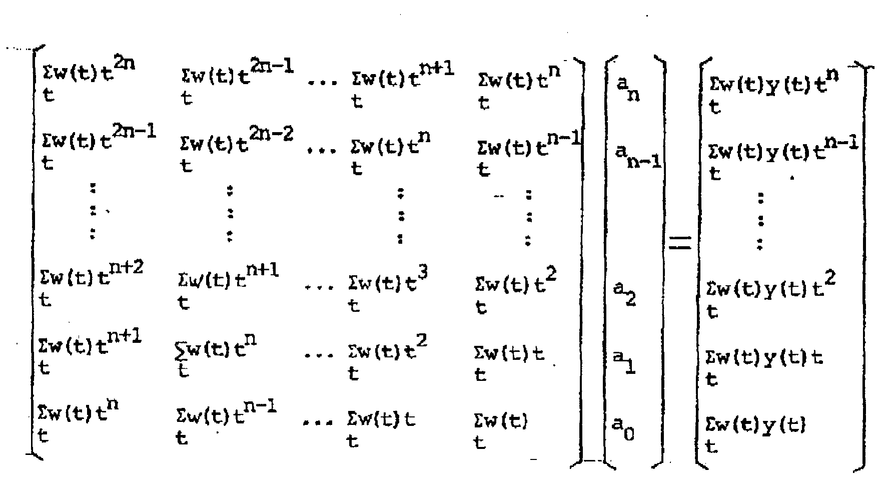

## Patentansprüche

1. Ultraschallanalysator zur Verwendung in einem Ultraschallinstrument, welches im Betrieb Ultraschallimpulse auf ein zu untersuchendes Medium richten und ein Echosignal y(t) der vom Medium reflektierten Impulse erfassen kann, wobei in diesem Instrument ein Analyseauswahlsignal w(t) abhängig von dem erfaßten Echosignal y(t) erzeugt wird, so daß das Analyseauswahlsignal w(t) während der Perioden, wo das Echosignal y(t) Rauschkomponenten enthält, einen ersten Wert und außerhalb solcher Perioden einen zweiten Wert annimmt und wobei der Analysator zur Bestimmung des Koeffizienten a1 einer das während eines Analyseintervalls der Dauer T gemessene Echosignal y(t) approximierenden Regressionsfunktion f(t) = a₁t + a₀ betreibbar ist, wobei das Analyseintervall der Dauer T auf eine gewählte Analysezeit, die relativ zu einer Bezugszeit gemessen ist, zentriert ist, und der Analysator aufweist:
eine erste Einrichtung (100), die zum Empfang des Analyseauswahlsignals w(t) und des Echosignals y(t) verbunden ist zur Erzeugung jeweiliger Ausgangssignale (A, B), die die Startzeit t₁ und die Endzeit t₂ des Analyseintervalls angeben, wenn eine Analysezeit gewählt wurde, wobei die erste Einrichtung (100) im Betrieb während des Empfangs des Echosignals y(t) die Gesamtzeitdauer aufeinanderfolgender Perioden bestimmt, wenn das Echosignal y(t) keine Rauschkomponenten enthält und von diesem aufeinanderfolgende Werte bezüglich der von der Bezugszeit an vergangenen Zeit t speichert und diese Werte, wenn eine Analysezeit ausgewählt ist, zur Bestimmung der Startzeit t₁ und der Endzeit t₂ so verwendet, daß die Gesamtdauer dieser Perioden zwischen t₁ und t und die Gesamtdauer dieser Perioden zwischen t und t₂ jeweils gleich T/2 sind;
eine zweite Einrichtung (110, 120, 130, 140), die zum Empfang des Analyseauswahlsignals w(t) und des Echosignals y(t) verbunden ist und im Betrieb während des Empfangs des Echosignals y(t) diese Signale w(t), y(t) zur Erzeugung eines Satzes von vier Integrationssignalen (I₀, I₁, I₂, I₃) verwendet, welche durch Akkumulieren jeweiliger Multiplikationssignale w(t)t, w(t)t², w(t)y(t)t, w(t)y(t) berechnet werden, wobei die Multiplikationssignale durch Multiplikation des Analyseauswahlsignals w(t) jeweils mit einem Signal eines Satzes von Multiplikatorsignalen erzeugt werden, welche aus einem die Zeit, die von der Bezugszeit vergangen ist, darstellenden Zeitsignal t, einem Quadratsignal t², das das Quadrat des Zeitsignals t darstellt, einem Produktsignal y(t)t, das die Multiplikation des Echosignals y(t) mit dem Zeitsignal darstellt und dem Echosignal y(t) bestehen;
eine dritte Einrichtung (111, 121, 131, 141), die zum Empfang des Ausgangssignals (A, B) der ersten Einrichtung verbunden ist, wobei die dritte Einrichtung (111, 121, 131, 141)im Betrieb aufeinanderfolgende Werte jedes der Integrationssignale (I₀, I₁, I₂, I₃) bezüglich der von der Bezugszeit vergangenen Zeit t speichert und auf den Empfang der Ausgangssignale hin an ihren jeweiligen Ausgängen (113, 123, 133, 143) Signale (X₁, X₂, X₃, X₄) erzeugt, die die Differenz zwischen den gespeicherten Werten zur Startzeit und zur Endzeit t₁, t₂ in bezug auf jedes der Integrationssignale (I₀, I₁, I₂, I₃) angeben; und
eine vierte Einrichtung (150), die zum Empfang der von den Ausgängen (113, 123, 133, 134) der dritten Einrichtung (111, 121, 131, 141) gelieferten Signale (X₁, X₂, X₃, X₄) verbunden ist und diese Signale zur Berechnung des Koeffizienten a1 der Regressionsfunktion f(t) mittels folgender Gleichung verwendet:$\text{a₁ =} \frac{\text{TΣw(t)y(t)t - Σw(t)y(t).Σw(t)t}}{\text{TΣw(t)t² - (Σw(t)t)²}}$

2. Ultraschallanalysator nach Anspruch 1, wobei die erste Einrichtung (100) aufweist: einen ersten Zähler (10) zum Zählen eines Taktsignals (CLK), wenn das Analyseauswahlsignal w(t) den zweiten Wert hat und zur Ausgabe eines Zusatzintegrationssignals (s) in Abhängigkeit vom Taktsignal (CLK) während eines Schreibmodus des Analysators; einen zweiten Zähler (14) zum Zählen des Taktsignals (CLK) und zur Ausgabe des Zeitsignals t; einen ersten Speicher (11) zum Speichern des Zusatzintegrationssignals (s) unter Verwendung des Zeitsignals t als Adresse; einen zweiten und dritten Speicher (12, 13) zum Speichern des Zeitsignals t als Daten unter Verwendung des Zusatzintegrationssignals (s) als Adresse; und eine erste und zweite Addierschaltung (16, 17) zur Ausgabe jeweils erster und zweiter Adressen (s-T/2; s+T/2) durch Verschieben einer Adresse (s), die vom ersten Speicher (11) während eines Lesemodus des Analysators mittels einer Adresse, die T/2 entspricht, ausgegeben wird; wobei der zweite Speicher (12) den Startzeitpunkt t₁ abhängig von der ersten Adresse (s-T/2) und der dritte Speicher (13) den Endzeitpunkt t₂ in Abhängigkeit von der zweiten Adresse (s+T/2) ausgeben.

3. Ultraschallanalysator nach Anspruch 2, der weiterhin eine Zeitsteuerschaltung (22) enthält zur Erzeugung: des Taktsignals (CLK); eines Lese/Schreib-Steuersignals (W/R) zur Umschaltung zwischen dem Schreibmodus und dem Lesemodus, wobei das Schreib/Lese-Steuersignal (W/R) parallel der dritten Einrichtung (111, 121, 131, 141) eingegeben wird; und eines Rücksetzsignals (CLR) zum Rücksetzen des ersten und zweiten Zählers (10, 14).

4. Ultraschallanalysator nach Anspruch 2 oder 3, wobei jeder erste, zweite und dritte Speicher (11, 12, 13) eine Tabelle zum Speichern des Zusatzintegrationssignals (s) und des Zeitsignals (t) aufweist, die die Adresse (t) des ersten Speichers (11) oder der Daten (t) des zweiten und dritten Speichers (12, 13) in Abszissenrichtung und die Daten (s) des ersten Speichers (11) oder die Adresse (s) des zweiten und dritten Speichers (12, 13) in Ordinatenrichtung beinhaltet.

5. Ultraschallanalysator nach irgend einem der vorangehenden Ansprüche, wobei die zweite Einrichtung (110, 120, 130, 140) vier Multiplikations/Integrationsschaltungen zur Erzeugung jeweils zugeordneter Integrationssignale (I₀, I₁, I₂, I₃), umfaßt, von denen jede Schaltung aufweist: eine Multiplikationsschaltung (23) zur Multiplikation des Analyseauswahlsignals w(t) mit einem zugeordneten der Multiplikatorsignale und zur Ausgabe eines Multiplikationswerts (R₂); eine Addierschaltung (241) zur Integration des Multiplikationswerts (R₂) und Ausgabe des Integrationssignals (I₀, I₁, I₂, I₃); und ein Register (242), um das Integrationssignal (I₀, I₁, I₂, I₃) zwischenzuspeichern.

6. Ultraschallanalysator nach irgend einem der vorangehenden Ansprüche, wobei die dritte Einrichtung (111, 121, 131, 141) vier Speichereinheiten zum Speichern aufeinanderfolgender Werte jeweils zugeordneter Integrationssignale (I₀, I₁, I₂, I₃) aufweist und jede Speichereinheit aufweist:
einen ersten und zweiten Speicher (25, 26), die mit der ersten Einrichtung (100) verbunden sind, um an ihren jeweiligen Adresseneingängen das Zeitsignal t während eines Schreibmodus des Analysators und jeweils eines der Ausgangssignale (A, B) während eines Lesemodus des Analysators zu empfangen, wobei der erste und zweite Speicher (25, 26) während des Schreibmodus zum Speichern aufeinanderfolgender Werte des Integrationssignals (I₀, I₁, I_{2,} I₃), die den an ihren Adresseneingängen gelieferten Adressen zugeordnet sind und in einem Lesemodus zur Ausgabe jeweiliger Posten solcher Daten betreibbar sind, die unter den Adressen gespeichert sind, welche ihren Adresseneingängen zur Verfügung gestellt werden;
eine Addierschaltung (27), die zum Empfang der vom ersten und zweiten Speicher (25, 26) ausgegebenen Daten verbunden ist und während des Lesemodus zur Erzeugung eines Signals (x₁, x₂, x₃, x₄) betreibbar ist, welches einen Differenzwert zwischen ihnen angibt; und
eine Dividierschaltung (28), die einen laufenden Mittelwert durch Division der Differenzwerte (x₁, x₂, x₃, x₄) durch die Dauer des Analyseintervalls T berechnet.

7. Ultraschallanalysator nach Anspruch 6, wobei der erste und zweite Speicher (25, 26) jeweils eine Tabelle aufweisen, die die Werte des Integrationssignals (I₀, I₁, I₂, I₃) speichert, wobei die Tabelle die Adresse des ersten und zweiten Speichers (25, 26) in Abszissenrichtung und die Daten des ersten und zweiten Speichers (25, 26) in Ordinatenrichtung enthält.

8. Ultraschallanalysator nach irgend einem der vorangehenden Ansprüche, wobei die von der dritten Einrichtung (111, 121, 131, 141) der vierten Einrichtung (150) zugeführten Signale (x₁ x₂, x₃, x₄) einen ersten, zweiten, dritten und vierten Differenzwert (x₁, x₂, x₃, x₄) angeben und die vierte Einrichtung (150) aufweist: eine Quadrierschaltung (52) zur Berechnung eines Quadratwerts des ersten Differenzwerts (x₁); eine Multiplizierschaltung (54) zur Multiplikation des ersten Differenzwerts (x₁) mit dem vierten Differenzwert (x₄); einen ersten Addierer (53) zur Addition eines Ausgangs der Quadrierschaltung (52) zum zweiten Differenzwert (x₂); einen zweiten Addierer (55) zur Addition eines Ausgangs der Multiplikationsschaltung (54) zum dritten Differenzwert (x₃) und eine Dividierschaltung (56) zur Berechnung des Koeffizienten a₁ der Regressionsfunktion f(t), durch Division eines Ausgangssignals des zweiten Addierers (55) durch ein Ausgangssignal des ersten Addierers (53).

9. Ultraschallanalysator nach irgend einem der vorangehenden Ansprüche, der weiterhin eine Quadrierschaltung (50) zur Erzeugung des Quadratsignals t² aus dem Zeitsignal t und eine Multiplikationsschaltung (51) aufweist, um das Produktsignal y(t)t durch Multiplikation des Zeitsignals t mit dem Echosignal y(t) zu erzeugen.

10. Ultraschallanalysator nach irgend einem der vorangehenden Ansprüche, wobei das Analyseauswahlsignal w(t) durch eine Analyseauswahlsignalerzeugungsschaltung erzeugt wird, die aufweist: eine Einhüllendenerfassungsschaltung (29), die eine Einhüllende aus dem Echosignal y(t) und eine logarithmische Kurve aus der Einhüllenden erzeugt; ein CFAR-System (30) zur Erzeugung eines CFAR-Signals, basierend auf der logarithmischen Kurve; einen ersten Komparator (31), der das CFAR-Signal mit einem unteren Grenzpegel vergleicht; einen zweiten Komparator (32), der das CFAR-Signal mit einem oberen Grenzpegel vergleicht; eine NICHT-Schaltung (33), die ein Ausgangssignal des zweiten Komparators (32) invertiert; und eine UND-Schaltung (34), die das Analyseauswahlsignal, basierend auf einem Ausgangssignal der NICHT-Schaltung (33) und einem Ausgangssignal des ersten Komparators (31), erzeugt.

11. Ultraschallanalysator zur Verwendung in einem Ultraschallinstrument, das im Betrieb Ultraschallimpulse auf ein zu untersuchendes Medium richten und ein Echosignal y(t) der vom Medium reflektierten Impulse erfassen kann, in welchem Instrument ein Analyseauswahlsignal w(t) abhängig vom erfaßten Echosignal y(t) so erzeugt wird, daß das Analyseauswahlsignal w(t) einen ersten Wert während der Perioden annimmt, wo das Echosignal y(t) Rauschkomponenten und einen zweiten Wert außerhalb solcher Perioden annimmt, wobei der Analysator zur Ermittlung der Koeffizienten a₁ bis aₙ einer Regressionsfunktion f(t)=aₙtⁿ + aₙ₋₁tⁿ⁻¹ + ... + a₁t + a₀ betreibbar ist, welche das während eines Analyseintervalls der Dauer T erfaßte Echosignal y(t) approximiert, wobei das Analyseintervall der Dauer T auf eine gewählte, relativ zu einer Bezugszeit gemessenen Analysezeit zentriert ist und wobei der Analysator aufweist:
eine erste Einrichtung (100), die zum Empfang des Analyseauswahlsignals w(t) und des Echosignals y(t) verbunden ist zur Erzeugung jeweiliger Ausgangssignale, die die Startzeit t₁ und die Endzeit t₂ des Analyseintervalls angeben, wenn eine Analysezeit gewählt wurde, wobei die erste Einrichtung im Betrieb während des Empfangs des Echosignals y(t) die Gesamtzeitdauer aufeinanderfolgender Perioden bestimmt, wenn das Echosignal y(t) keine Rauschkomponenten enthält und von diesem aufeinanderfolgende Werte (s) bezüglich der von der Bezugszeit an vergangenen Zeit t speichert und diese Werte (s), wenn eine Analysezeit ausgewählt ist, zur Bestimmung der Startzeit t₁ und der Endzeit t₂ so verwendet, daß die Gesamtdauer dieser Perioden zwischen t₁ und t und die Gesamtdauer dieser Perioden zwischen t und t₂ jeweils gleich T/2 sind;
eine zweite Einrichtung (110, 120, 130, 140, ...), die zum Empfang des Analyseauswahlsignals w(t) und des Echosignals y(t) verbunden und im Betrieb während des Empfangs des Echosignals y(t) diese Signale w(t), y(t) zur Erzeugung eines Satzes von Integrationssignalen (I₀, I₁, I₂, I₃, ...), die durch Akkumulieren jeweiliger Multiplikationssignale berechnet werden, wobei die Multiplikationssignale durch Multiplikation des Analyseauswahlsignals w(t) mit jeweiligen Signalen eines Satzes von Multiplikatorsignalen erzeugt werden, die aus einem Zeitsignal t, das die Zeit darstellt, die von der Bezugszeit an vergangen ist, zur Erzeugung von Potenzsignalen t^{k}, wobei k = 0 bis n, die die k-te Potenz des Zeitsignals t angeben, eines Produktsignals y(t)tk, das die Multiplikation des Echosignals y(t) mit den Potenzsignalen t^{k} darstellt, und zur Erzeugung des Echosignals y(t) verwendet;
eine dritte Einrichtung (111, 121, 131, 141, ....), die zum Empfang der Ausgangssignale (A, B) der ersten Einrichtung (100) verbunden ist, und die im Betrieb aufeinanderfolgende Werte jedes der Integrationssignale (I₀, I₁, I₂, I₃, ....) in bezug auf die von der Referenzzeit an vergangenen Zeit t speichert, und die auf den Empfang der Ausgangssignale (A, B) hin an ihren jeweiligen Ausgängen (113, 123, 133, 143, ....) Signale (x₁, x₂, x₃, x₄, ....) liefert, die die Differenz zwischen den gespeicherten Werten am Startzeitpunkt und Endzeitpunkt (t₁, t₂) in bezug auf jedes der Integrationssignale (I₀, I₁, I₂, I₃, ....) angeben; und
eine vierte Einrichtung (150), die zum Empfang der an den Ausgängen der dritten Einrichtung (111, 121, 131, 141, ....) erzeugten Signale (x₁, x₂, x₃, x₄, ....) verbunden ist und diese Werte zur Berechnung der Koeffizienten a₁ bis aₙ der Regressionsfunktion f(t) verwendet, indem sie folgende Matrix löst:

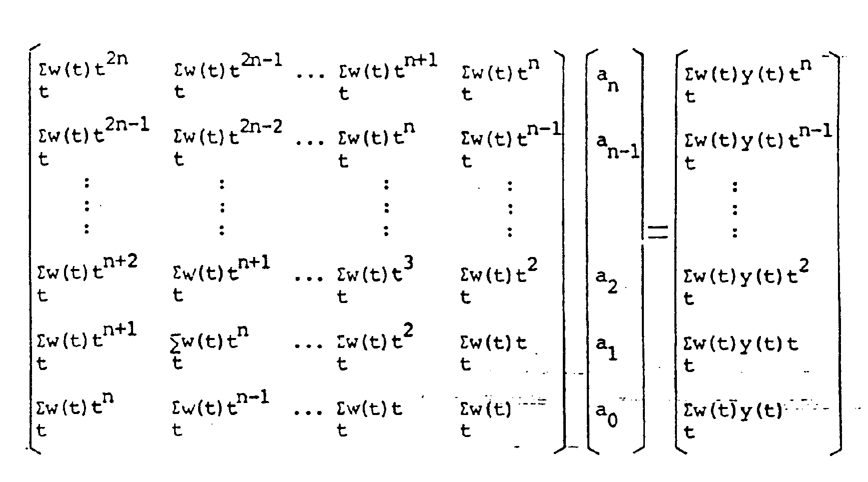

## Revendications

1. Analyseur d'ultrasons destiné à être utilisé dans un instrument à ultrasons fonctionnant pour diriger des impulsions ultrasonores au niveau d'un milieu qui doit être étudié et pour détecter un signal d'écho y(t) de ces impulsions réfléchies depuis ledit milieu, instrument dans lequel un signal de sélection d'analyse w(t) est généré en fonction du signal d'écho détecté y(t) de telle sorte que le signal de sélection d'analyse w(t) présente une première valeur pendant les périodes pendant lesquelles le signal d'écho y(t) contient des composantes de bruit et une seconde valeur à l'extérieur de ces périodes, lequel analyseur peut fonctionner pour déterminer le coefficient a₁ d'une fonction de régression f(t) = a₁t + a₀ permettant d'approximer le signal d'écho y(t) détecté pendant un intervalle d'analyse d'une durée T centré sur un instant d'analyse sélectionné mesuré par rapport à un instant de référence, et comprenant :
un premier moyen (100) connecté pour recevoir ledit signal de sélection d'analyse w(t) et le signal d'écho y(t) pour produire des signaux de sortie respectifs (A, B) indicatifs d'un instant de début t₁ et d'un instant de fin t₂ dudit intervalle d'analyse lorsqu'un instant d'analyse a été sélectionné, lequel premier moyen (100) peut fonctionner pendant la réception dudit signal d'écho y(t) pour déterminer la durée totale de périodes successives lorsque le signal d'écho y(t) ne contient pas des composantes de bruit et pour stocker des valeurs successives (s) de celui-ci par rapport au temps t écoulé depuis ledit instant de référence et pour utiliser ces valeurs lorsqu'un instant d'analyse est sélectionné pour déterminer l'instant de début t₁ et l'instant de fin t₂ de telle sorte que la durée totale des périodes qui se situent entre t₁ et t et que la durée totale des périodes qui se situent entre t et t₂ soient chacune égale à T/2;
un second moyen (110, 120, 130, 140) connecté pour recevoir ledit signal de sélection d'analyse w(t) et ledit signal d'écho y(t) et fonctionnant, pendant la réception dudit signal d'écho y(t), pour utiliser ces signaux w(t), y(t) pour générer un jeu de quatre signaux d'intégration (I₀, I₁, I₂, I₃) calculés en accumulant des signaux de multiplication respectifs w(t)t, w(t)t², w(t)y(t)t, w(t)y(t), lesquels signaux de multiplication sont produits en multipliant ledit signal de sélection d'analyse w(t) par certains respectifs d'un jeu de signaux de multiplieur constitués par un signal de temps t représentant le temps qui s'est écoulé depuis l'instant de référence, par un signal carré t2 représentant le carré dudit signal de temps t, par un signal de produit y(t)t représentant la multiplication dudit signal d'écho y(t) par ledit signal de temps t, et par ledit signal d'écho y(t) ;
un troisième moyen (111, 121, 131, 141) connecté pour recevoir les signaux de sortie (A, B) dudit premier moyen (100), lequel troisième moyen (111, 121, 131, 141) peut fonctionner pour stocker des valeurs successives de chacun desdits signaux d'intégration (I₀, I₁, I₂, I₃) par rapport au temps t écoulé depuis ledit instant de référence et suite à la réception desdits signaux de sortie (A, B), pour produire au niveau de sorties respectives (113, 123, 133, 143) de celui-ci des signaux (x₁, x₂, x₃,x₄) indicatifs de la différence entre les valeurs stockées aux instants de début et de fin t₁, t₂ par rapport à chacun desdits signaux d'intégration (I₀, I₁, I₂, I₃) ; et
un quatrième moyen (150) connecté pour recevoir les signaux (x₁, x₂, x₃, x₄) produits au niveau desdites sorties (113, 123, 133, 143) du troisième moyen (111, 121, 131, 141) et pour utiliser ces signaux afin de calculer le coefficient a₁ de ladite fonction de régression f(t) à partir de l'équation :$\text{a₁ =} \frac{\text{TΣw(t)y(t)t - Σw(t)y(t).Σw(t)t}}{\text{TΣw(t)t² - (Σw(t)t)²}} \text{.}$

2. Analyseur d'ultrasons selon la revendication 1, dans lequel ledit premier moyen (100) comprend : un premier compteur (10) pour compter un signal d'horloge (CLK) lorsque ledit signal de sélection d'analyse w(t) présente ladite seconde valeur et pour émettre en sortie un signal d'intégration auxiliaire (s) en réponse audit signal d'horloge (CLK) pendant un mode écriture de l'analyseur ; un second compteur (14) pour compter ledit signal d'horloge (CLK) et pour émettre en sortie ledit signal de temps t une première mémoire (11) pour stocker ledit signal d'intégration auxiliaire (s) en utilisant ledit signal de temps t en tant qu'adresse des seconde et troisième mémoires (12, 13) pour stocker ledit signal de temps t en tant que données en utilisant ledit signal d'intégration auxiliaire (s) en tant qu'adresse et des premier et second circuits additionneurs (16, 17) pour émettre en sortie des première et seconde adresses respectives (s-T/2 ; s+T/2) en décalant une adresse (s) émise en sortie par ledit premier moyen de mémoire (11) pendant un mode lecture de l'analyseur d'une adresse correspondant à T/2 ; ladite seconde mémoire (12) émettant en sortie ledit instant de début t₁ en réponse à ladite première adresse (s-T/2) et ladite troisième mémoire (13) émettant en sortie ledit instant de fin t₂ en réponse à ladite seconde adresse (S+T/2).

3. Analyseur d'ultrasons selon la revendication 2, comprenant en outre un circuit de commande de cadencement (22) pour générer ledit signal d'horloge (CLK), un signal de commande d'écriture/lecture (W/R) pour réaliser une commutation entre ledit mode écriture et ledit mode lecture, ledit signal de commande d'écriture/lecture (W/R) étant entré en parallèle sur ledit troisième moyen (111, 121, 131, 141) ; et un signal de remise à zéro (CLR) pour remettre à l'état initial lesdits premier et second compteurs (10, 14).

4. Analyseur d'ultrasons selon la revendication 2 ou 3, dans lequel chacune desdites première, seconde et troisième mémoires (11, 12, 13) comprend une table pour stocker ledit signal d'intégration auxiliaire (s) et ledit signal de temps t, ladite table comportant l'adresse (t) de ladite première mémoire (11) ou les données (t) desdites seconde et troisième mémoires (12, 13) suivant une direction d'abscisse et comportant les données (s) de ladite première mémoire (11) ou l'adresse (s) desdites seconde et troisième mémoires (12, 13) suivant une direction d'ordonnée.

5. Analyseur d'ultrasons selon l'une quelconque des revendications précédentes, dans lequel ledit second moyen (110, 120, 130, 140) comprend quatre circuits de multiplication/intégration pour produire certains respectifs desdits signaux d'intégration (I₀, I₁, I₂, I₃), chacun de ces circuits comprenant : un circuit de multiplication (23) pour multiplier ledit signal de sélection d'analyse w(t) par l'un associé desdits signaux de multiplieur et pour émettre en sortie une valeur de multiplication (R₂) ; un circuit additionneur (241) pour intégrer ladite valeur de multiplication (R₂) et pour émettre en sortie ledit signal d'intégration (I₀, I₁, I₂, I₃) ; et un registre (242) pour stocker temporairement ledit signal d'intégration (I₀, I₁, I₂, I₃).

6. Analyseur d'ultrasons selon l'une quelconque des revendications précédentes, dans lequel ledit troisième moyen (111, 121, 131, 141) comprend quatre unités de stockage pour stocker des valeurs successives de certains associés desdits signaux d'intégration (I₀, I₁, I₂, I₃), chaque unité de stockage comprenant :
des première et seconde mémoires (25, 26) connectées audit premier moyen (100) de manière à recevoir au niveau d'entrées d'adresse respectives de celui-ci ledit signal de temps t pendant un mode écriture de l'analyseur et certains respectifs desdits signaux de sortie (A, B) pendant un mode lecture de l'analyseur, lesdites première et seconde mémoires (25, 26) pouvant fonctionner pendant un mode écriture pour stocker des valeurs successives du signal d'intégration (I₀, I₁, I₂, I₃) associées au niveau d'adresses produites au niveau de leurs entrées d'adresse et pendant un mode lecture pour émettre en sortie des éléments respectifs de ces données stockées au niveau des adresses produites au niveau de leurs entrées d'adresse ;
un circuit additionneur (27) connecté pour recevoir les données émises en sortie par lesdites première et seconde mémoires (25, 26) et pouvant fonctionner pendant ledit mode lecture pour générer un signal (x₁, x₂, x₃, x₄) indicatif d'une valeur de différence entre ; et
un circuit diviseur (28) pour calculer une valeur moyenne mobile en divisant ladite valeur de différence (x₁, x₂, x₃, x₄) par la durée dudit intervalle d'analyse T.

7. Analyseur d'ultrasons selon la revendication 6, dans lequel chacune desdites première et seconde mémoires (25, 26) comprend une table pour stocker lesdites valeurs du signal d'intégration (I₀, I₁, I₂, I₃), ladite table comportant l'adresse desdites première et seconde mémoires (25, 26) suivant une direction d'abscisse et comportant les données desdites première et seconde mémoires (25, 26) suivant une direction d'ordonnée.

8. Analyseur d'ultrasons selon l'une quelconque des revendications précédentes, dans lequel les signaux (x₁, x₂, x₃, x₄) appliqués audit quatrième moyen (150) par ledit troisième moyen (111, 121, 131, 141) sont indicatifs de première, seconde, troisième et quatrième valeurs de différence (x₁, x₂, x₃, x₄) et ledit quatrième moyen (150) comprend : un circuit d'élévation au carré (52) pour calculer une valeur élevée au carré de ladite première valeur de différence (x₁) ; un circuit de multiplication (54) pour multiplier ladite première valeur de différence (x₁) par ladite quatrième valeur de différence (x₄) ; un premier additionneur (53) pour additionner une sortie dudit circuit d'élévation au carré (52) à ladite seconde valeur de différence (x₂) un second additionneur (55) pour additionner une sortie dudit circuit de multiplication (54) à ladite troisième valeur de différence (x₃) et un circuit diviseur (56) pour calculer le coefficient a₁ de ladite fonction de régression f(t) en divisant une sortie dudit second additionneur (55) par une sortie dudit premier additionneur (53).

9. Analyseur d'ultrasons selon l'une quelconque des revendications précédentes, comprenant en outre un circuit d'élévation au carré (50) pour générer ledit signal élevé au carré t₂ à partir dudit signal d'instant t et un circuit de multiplication (51) pour générer ledit signal de produit y(t)t en multipliant ledit signal d'instant t par ledit signal d'écho y(t).

10. Analyseur d'ultrasons selon l'une quelconque des revendications précédentes, dans lequel ledit signal de sélection d'analyse w(t) est généré par un circuit de génération de signal de sélection d'analyse comprenant un circuit de détection d'enveloppe (29) pour obtenir une enveloppe à partir dudit signal d'écho y(t) et pour obtenir une courbe logarithmique à partir de ladite enveloppe ; un système CFAR (30) pour générer un signal CFAR sur la base de ladite courbe logarithmique un premier comparateur (31) pour comparer ledit signal CFAR à un niveau de limite inférieure un second comparateur (32) pour comparer ledit signal CFAR à un niveau de limite supérieure ; un circuit NON (33) pour inverser une sortie dudit second comparateur (32); et un circuit ET (34) pour générer ledit signal de sélection d'analyse sur la base d'une sortie dudit circuit NON (33) et d'une sortie dudit premier comparateur (31).

11. Analyseur d'ultrasons destiné à être utilisé dans un instrument à ultrasons fonctionnant pour diriger des impulsions ultrasonores au niveau d'un milieu qui doit être étudié et pour détecter un signal d'écho y(t) de ces impulsions réfléchies depuis ledit milieu, instrument dans lequel un signal de sélection d'analyse w(t) est généré en fonction du signal d'écho détecté y(t) de telle sorte que le signal de sélection d'analyse w(t) présente une première valeur pendant les périodes pendant lesquelles le signal d'écho y(t) contient des composantes de bruit et une seconde valeur à l'extérieur de ces périodes, lequel analyseur peut fonctionner pour déterminer les coefficients a₁ à aₙ d'une fonction de régression f(t) = aₙtⁿ + aₙ₋₁tⁿ⁻¹ + a₁t + a₀ permettant d'approximer le signal d'écho y(t) détecté pendant un intervalle d'analyse d'une durée T centré sur un instant d'analyse sélectionné mesuré par rapport à un instant de référence, et comprenant :
un premier moyen (100) connecté pour recevoir ledit signal de sélection d'analyse w(t) et le signal d'écho y(t) pour produire des signaux de sortie respectifs (A, B) indicatifs d'un instant de début t₁ et d'un instant de fin t₂ dudit intervalle d'analyse lorsqu'un instant d'analyse a été sélectionné, lequel premier moyen (100) peut fonctionner pendant la réception dudit signal d'écho y(t) pour déterminer la durée totale de périodes successives lorsque le signal d'écho - y(t) ne contient pas des composantes de bruit et pour stocker des valeurs successives (s) de celui-ci par rapport à l'instant t écoulé depuis ledit instant de référence et pour utiliser ces valeurs (s) lorsqu'un instant d'analyse est sélectionné pour déterminer l'instant de début t₁ et l'instant de fin t₂ de telle sorte que la durée totale des périodes qui se situent entre t₁ et t et que la durée totale des périodes qui se situent entre t et t₂ soient chacune égale à T/2 ;
un second moyen (110, 120, 130, 140,...) connecté pour recevoir ledit signal de sélection d'analyse w(t) et ledit signal d'écho y(t) et fonctionnant, pendant la réception dudit signal d'écho y(t), pour utiliser ces signaux w(t), y(t) pour générer un jeu de quatre signaux d'intégration (I₀, I₁, I₂, I₃,...) calculés en accumulant des signaux de multiplication respectifs, lesquels signaux de multiplication sont produits en multipliant ledit signal de sélection d'analyse w(t) par certains respectifs d'un jeu de signaux de multiplieur constitués par un signal de temps t représentant le temps qui s'est écoulé depuis l'instant de référence, par des signaux de puissance t^{k} où k = 0 à n représentant la puissance k-ième dudit signal de temps t, par un signal de produit y(t)t^{k} représentant la multiplication dudit signal d'écho y(t) par lesdits signaux de puissance t^{k}, et par ledit signal d'écho y(t) ;
un troisième moyen (111, 121, 131, 141,...) connecté pour recevoir les signaux de sortie (A, B) dudit premier moyen (100), lequel troisième moyen (111, 121, 131, 141,...) peut fonctionner pour stocker des valeurs successives de chacun desdits signaux d'intégration (I₀, I₁ , I₂, I₃,...) par rapport au temps t écoulé depuis ledit instant de référence et suite à la réception desdits signaux de sortie (A, B), pour produire au niveau de sorties respectives (113, 123, 133, 143,...) de celui-ci des signaux (x₁, x₂, x₃, x₄,...) indicatifs de la différence entre les valeurs stockées aux instants de début et de fin t₁, t₂ par rapport à chacun desdits signaux d'intégration (I₀, I₁, I₂, I₃,...); et
un quatrième moyen (150) connecté pour recevoir les signaux (x₁, x₂, x₃, x₄,...) produits au niveau desdites sorties (113, 123, 133, 143) du troisième moyen (111, 121, 131, 141,...) et pour utiliser ces valeurs afin de calculer les coefficients a₁ à aₙ de ladite fonction de régression f(t) en résolvant la matrice :
